# EUROPEAN PATENT APPLICATION

(11) **EP 0 688 761 A2**
(43) Date of publication of application: **27.12.1995**
(21) Application number: 95830209.3
(22) Date of filing: 18.05.1995
(51) Int. Cl.: C07C 229/22, A61K 31/195

(54) **L-carnitine salt and cosmetic and pharmaceutical compositions containing same for treating dermatoses**

(30) Priority: 20.06.1994 IT RM940396
(71) Applicant: AVANTGARDE S.p.A., I-00040 Pomezia(Rome) (IT)
(72) Inventor: Cavazza, Claudio, I-00186 Rome (IT); Cavazza, Paolo, I-00144 Rome (IT)
(74) Representative: Fassi, Aldo

(57) **Abstract**

L-carnitine glycolate and its use for producing cosmetic and pharmaceutical compositions suitable to be topically applied for treating dermatoses, are disclosed.

## Description

The present invention relates to a novel L-carnitine salt and the use thereof for producing cosmetic and pharmaceutical compositions which contain such salt, suitable to be topically applied for the treatment of dermatoses.

This novel L-carnitine salt is L-carnitine glycolate of formula (I)

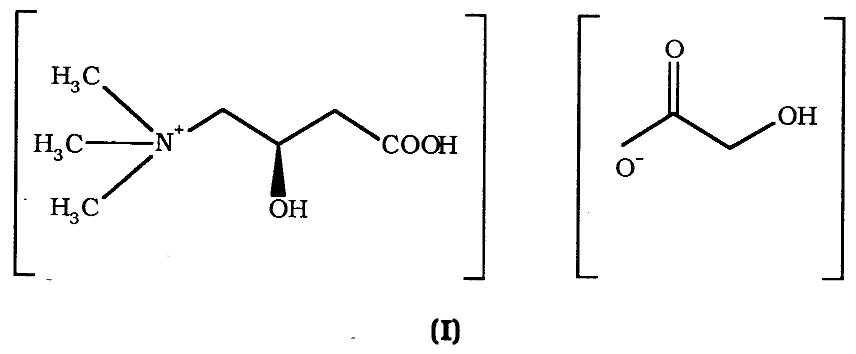

L-carnitine glycolate is a highly stable and non-hygroscopic salt. This is quite surprising since glycolic acid is somewhat hygroscopic. Surprising as well is the activity of this novel salt as dermatologic drug if one considers that glycolic acid is mildly irritant to skin and mucous membranes.

In the following example the preparation of L-carnitine glycolate is described.

### EXAMPLE

**Preparation of L-carnitine glycolate (ST 1136).**

L-carnitine inner salt (16.1 g; 0.1 moles) was dissolved in 100 mL H₂O. Glycolic acid (7.6 g; 0.1 moles) was added to the solution.

The resulting solution was concentrated under vacuum at 40°C. The residue was taken up with isopropanol and the solid residue filtered off. 23 g were obtained.

The compound was crystallized from hot isopropanol.
M.P. = 112.3°C (DSC)

| Elementary analysis C₉H₁₇NO₆ | | | |
|---|---|---|---|
| | C% | H% | N% |
| Calculated | 45.95 | 7.28 | 5.95 |
| Found | 46.17 | 8.46 | 6.06 |

Water content 0.3%
pH= 3.9
**[α]**$\frac{\text{25}}{\text{D}}$ = -19.9° (c=1%, H₂O)
HPLC
Column: µ Bondapack-NH₂ (10 µm) 300 mm x 3.9 mm
Temperature: 30°C
Mobile phase: CH₃CN/KH₂PO₄ mM pH 5.2 65/35
Flow rate: 1.0 ml/min
Glycolic acid Rₜ = 4.91 min 31.6%
L-carnitine Rₜ = 8.99 min 68.4%
NMR D₂O δ 4.5-4.4(1H,m,CHOH); 3.9(2H,s,CH₂OH); 3.3(2H,m,CH₂N+); 3.1(9H,s,(CH₃)₃N+); 2.4(2H,dd,CH₂COO)

L-carnitine glycolate is used as active ingredient for producing dermatologic and cosmetic compositions suitable to be topically applied.

The dermatoses which are suitably treated with the compositions of the present invention are in particular ichthyosis, psoriasis and those dermatoses which are induced by a defective keratinization, such as dandruff, acne and palmar and plantar hyperkeratosis.

Ichthyosis is a dermatosis characterized by generalized dryness, harshness and scaling of the skin. It may occur as a hereditary disease present at birth, or as a metabolic disorder associated with hypothyroidism or with the intake of drugs (such as butyrophenols) inhibiting lipid synthesis, or as a paraneoplastic syndrome, manifestation of a tumor process involving internal organs.

Xeroderma, the mildest form of ichthyosis is neither congenital nor associated with systemic abnormalities.

It usually occurs on the lower legs of middle-aged or older patients, most often in cold weather and in patients who bathe frequently. There may be mild to moderate itching and an associated dermatitis due to detergents or other irritants.

The inherited ichthyoses, all characterized by excessive accumulation of scale on the skin surface, are classified according to clinical, genetic, and histologic criteria.

Known treatments of any form of ichthyosis comprise topically applying to the skin hydrating emollients. Furthermore, salicylic acid or vitamin A-containing ointments have been widely used.

A keratolytic agent particularly effective in removing the scales in ichthyosis vulgaris, lamellar ichthyosis and sex-linked ichthyosis contains 6% salicylic acid in a gel composed of propylene glycol, ethanol, hydroxypropylene cellulose and water.

Further known drugs for the treatment of this disorder include: 50% propylene glycol in water, hydrophilic petrolatum and water (in equal parts), and cold cream.

In lamellar ichthyosis, 0.1% tretinoin (vitamin A acid: retinoic acid) cream has been utilized.

None of these treatments has been found satisfactorily effective.

Hyperkeratosis is a thickening of the stratum corneum of the skin.

The treatment of choice is the topical application of drugs containing urea, propylene glycol or salicylic acid. Also in this case, none of the known treatment has proved to be satisfactorily effective.

It has now been found that the compound of the present invention, when topically applied as solutions, lotions, creams or ointments containing from 0.01% to 20%, preferably from 1% to 15% and most preferably from 2 to 10% by weight of the compound, is potently effective in achieving complete remission of ichthyotic conditions in humans and in healing psoriasis and those disorders brought about by an altered keratinization, such as dandruff, acne and palmar and plantar hyperkeratosis.

It has also been found that, if the solutions, creams or ointments of the invention are applied regularly on a daily basis, within about two to three weeks the affected skin areas will return to normal conditions.

In order to prepare the compositions of this invention, L-carnitine glycolate is first preferably dissolved in water or ethanol. The solution thus prepared may be admixed in the conventional manner with commonly available ointment bases such as hydrophilic ointments (USP) or petrolatum (USP).

The water or ethanol used to dissolve the compound of this invention may range in concentration of from 1 to 30%, by volume, of the total composition.

The compound of this invention may also be formulated in a solution or lotion form.

For instance, the salt is dissolved directly in a mixture of water, ethanol and propylene glycol (40:40:20 by weight).

Some examples of formulation are hereinbelow described:

### Formulation 1: 5% solution

5 grams of the salt were dissolved in 5 mL of water and the resulting solution admixed with 40 mL of ethanol and 20 mL of propylene glycol. Sufficient water was added to make 100 mL of formulation.

### Formulation 2: 5% ointment

5 grams of the salt were admixed with 95 grams of USP grade hydrophilic ointment, until a uniform consistency was obtained.

### CLINICAL TESTS

**(a) Treatment of ichthyosis.**
   Seven patients with ichthyosis were instructed to first wet their body by taking a shower and then to apply to the lesions a thin film of the 5% ointment prepared according to the above-mentioned Formulation 2. Topical applications were continued twice daily for several weeks. Generally, the affected skin became less scaly and felt smoother after two weeks of treatment. The sites of the lesions, devoid of any scales, usually reached an improved state comparable to normal appearing skin within two to four weeks after initial treatment.
   Once a normal appearing skin was restored, it remained improved for from several weeks to several months, varying from patient to patient, without further application of the ointment. It is, however, necessary to continue the application of the ointment in order to maintain the skin free from recurrence of the overt disease.
   The ointment in a concentration of 10% was similarly tested, and daily applications were found to be sufficient to clear the lesions and restore the skin to normal texture and moisture conditions.
**(b) Treatment of palmar and plantar hyperkeratosis.**
   Six patients with palmar and plantar hyperkeratosis secondary to chronic eczema or chronic friction were instructed to apply the above 5% solution of Formulation 1 topically, twice daily, to affected areas. All patients showed a substantial improvement after 2 weeks of treatment.
**(c) Treatment of dandruff.**
   Five patients with severe dandruff problems were instructed to rub into the scalp the above 5% solution twice weekly. This topically applied solution prevented all signs of dandruff, i.e., formation of scales on the scalp in all five patients. Relief was observed within about a week in each case and normal skin conditions were observed to be maintained for at least one to two weeks after treatment was terminated.

## Claims

1. L-carnitine glycolate of formula (I)

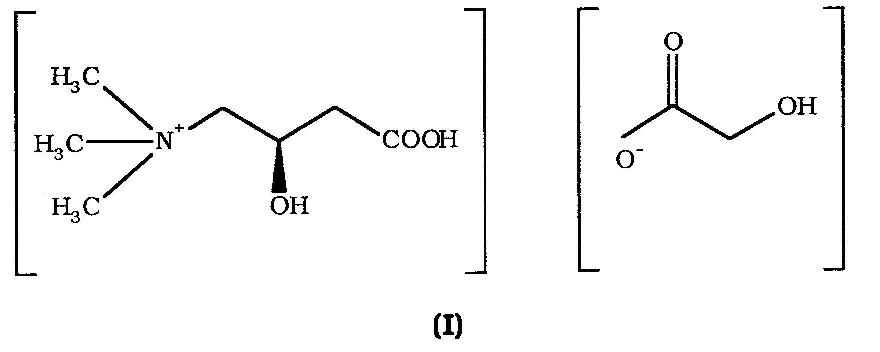

2. A pharmaceutical composition suitable to be topically applied for treating dermatoses, which comprises the compound of formula (I) as active ingredient and a pharmacologically acceptable excipient therefor.

3. The composition of claim 2 for treating ichthiosis and psoriasis.

4. The composition of claim 2 for treating dermatoses brought about by defective keratinization.

5. The composition of claim 4 for treating dandruff, acne and palmar and plantar hyperkeratosis.

6. The composition of anyone of the claims 2-5 in the form of solution, lotion, ointment or cream.

7. The composition of claim 6 which comprises from 0.01% to 20%, preferably from 1% to 15%, most preferably from 2% to 10% by weight, of the compound of formula (I).

8. A cosmetic composition which comprises the compound of formula (I) as active ingredient and a cosmetically acceptable excipient therefor.
